# EUROPÄISCHE PATENTSCHRIFT

(11) **EP 3 800 184 B1**
(45) Veröffentlichungstag und Bekanntmachung des Hinweises auf die Patenterteilung: **03.04.2024**
(21) Anmeldenummer: 20195764.4
(22) Anmeldetag: 11.09.2020
(51) Int. Cl.: C07D 317/30, A01N 35/02, A01N 49/00, A61L 9/01, C11D 3/50

(54) **ACETAL-/KETAL-BASIERTE DUFT- UND INSEKTENABWEHRMITTELVORLÄUFERVERBINDUNGEN**
ACETAL / KETAL-BASED FRAGRANCE AND INSECT REPELLENT PRECURSOR COMPOUNDS
COMPOSÉS PRÉCURSEURS D'AGENT ODORIFÉRANT ET INSECTIFUGE À BASE D'ACÉTAL / DE CÉTAL

(30) Priorität: 24.09.2019 DE 102019125579
(43) Veröffentlichungstag der Anmeldung: 07.04.2021
(73) Patentinhaber: Henkel AG & Co. KGaA, 40589 Düsseldorf (DE)
(72) Erfinder: SCHAEFER, Sascha, 40822 Mettmann (DE); BRUESS, Linda, 48149 Münster (DE)
(74) Vertreter: Viering, Jentschura & Partner mbB Patent- und Rechtsanwälte

(56) Entgegenhaltungen:
- EP-A1- 1 375 465
- WO-A1-97/34986
- WO-A1-2013/170778
- WO-A1-2018/124148
- WO-A2-2019/043587
- DE-A1-102016 223 412
- TORSSELL STAFFAN ET AL: "1,3-Dipolar Cycloadditions of Carbonyl Ylides to Aldimines: Scope, Limitations and Asymmetric Cycloadditions", ADVANCED SYNTHESIS & CATALYSIS, Bd. 348, Nr. 16-17, 1. November 2006 (2006-11-01), Seiten 2421-2430, XP055778829, DE ISSN: 1615-4150, DOI: 10.1002/adsc.200600324
- MORI ATSUNORI ET AL: "Asymmetric Simmons-Smith Reactions using Homochiral Protecting Groups", TETRAHEDRON, Bd. 42, Nr. 23, 1. Januar 1986 (1986-01-01), Seiten 6447-6458, XP055778874,
- ASHUTOSH V. BEDEKAR ET AL: "Investigation of the Effects of the Structure and Chelate Size of Bis-oxazoline Ligands in the Asymmetric Copper-Catalyzed Cyclopropanation of Olefins:? Design of a New Class of Ligands", THE JOURNAL OF ORGANIC CHEMISTRY, Bd. 62, Nr. 8, 1. April 1997 (1997-04-01), Seiten 2518-2526, XP055006366, ISSN: 0022-3263, DOI: 10.1021/jo962021a
- TSUZUKI YOJIRO: "Synthesis. of Cyclic Derivatives of Tartaric Acid by Condensing Alkyl d-Tartrates with Aliphatic Ketones. (Optical Activity and Chemical Structure in Tartaric Acid. V)", BULLETIN OF THE CHEMICAL SOCIETY OF JAPAN, Bd. 14, Nr. 1, 1. Januar 1939 (1939-01-01) , Seiten 19-22, XP055778962,
- NAHM MARY R. ET AL: "Metallophosphite-Catalyzed Asymmetric Acylation of [alpha],[beta]-Unsaturated Amides", JOURNAL OF THE AMERICAN CHEMICAL SOCIETY, Bd. 128, Nr. 8, 1. Januar 2006 (2006-01-01), Seiten 2751-2756, XP055779014, US ISSN: 0002-7863, DOI: 10.1021/ja056018x Gefunden im Internet: URL:https://pubs.acs.org/doi/pdf/10.1021/j a056018x> & Nahm Mary R ET AL: "Supporting Information Metallophosphite-Catalyzed Asymmetric Acylation of [alpha],[beta]-Unsaturated Amides", , 2. August 2006 (2006-08-02), Seiten S1-S5, XP055779037, Gefunden im Internet: URL:https://pubs.acs.org/doi/pdf/10.1021/j a056018x [gefunden am 2021-02-23]
- SEEBACH D ET AL: "CHIRALE ALKOXYTITAN(IV)-KOMPLEXE FUER ENANTIOSELEKTIVE NUCLEOPHILE ADDITIONEN AN ALDEHYDE UND ALS LEWIS-SAEUREN IN DIELS-ALDER-REAKTIONEN//CHIRAL ALKOXYTITANIUM(IV) COMPLEXES FOR ENANTIOSELECTIVE NUCLEOPHILIC ADDITIONS TO ALDEHYDE AND AS LEWIS ACIDS IN D", HELVETICA CHIMICA ACTA, VERLAG HELVETICA CHIMICA ACTA, Bd. 70, Nr. 4, 8. Juli 1987 (1987-07-08), Seiten 954-974, XP009065937, ISSN: 0018-019X, DOI: 10.1002/HLCA.19870700406
- INCH T. D. ET AL: "Structural assignments of some 2,2,4-trisubstituted 1,3-dioxolans", JOURNAL OF THE CHEMICAL SOCIETY, SECTION C: ORGANIC CHEMISTRY.>6015C, Nr. 2, 1. Januar 1970 (1970-01-01), Seite 263, XP055778764, GB ISSN: 0022-4952, DOI: 10.1039/j39700000263
- SORLIN ALEXANDRE M. ET AL: "The Role of Trichloroacetimidate To Enable Iridium-Catalyzed Regio- and Enantioselective Allylic Fluorination: A Combined Experimental and Computational Study", JOURNAL OF THE AMERICAN CHEMICAL SOCIETY, Bd. 141, Nr. 37, 22. August 2019 (2019-08-22), Seiten 14843-14852, XP055779209, US ISSN: 0002-7863, DOI: 10.1021/jacs.9b07575 Gefunden im Internet: URL:https://pubs.acs.org/doi/pdf/10.1021/j acs.9b07575> & Sorlin Alexandre M ET AL: "Supporting Information The Role of Trichloroacetimidate to Enable Iridium-Catalyzed Regio-and Enantioselective Allylic Fluorination: A Combined Experimental and Computational Study General Procedures & Analytical Data Crystal Data Computational Data HPLC Traces References NMR Data", , 22. August 2019 (2019-08-22), Seiten S1-S12, XP055779225, Gefunden im Internet: URL:https://pubs.acs.org/doi/suppl/10.1021 /jacs.9b07575/suppl_file/ja
- SOHN JEONG-HUN ET AL: "Total Synthesis of Mycalamide A", JOURNAL OF THE AMERICAN CHEMICAL SOCIETY, Bd. 127, Nr. 20, 1. Mai 2005 (2005-05-01), Seiten 7290-7291, XP055778768, US ISSN: 0002-7863, DOI: 10.1021/ja050728l
- BLICKE F F ET AL: "Antispasmodics. XI. Basic 1,3-Dioxolanes", JOURNAL OF THE AMERICAN CHEMICAL SOCIETY, Bd. 74, 1. Januar 1952 (1952-01-01), Seiten 2613-2615, XP055779285, Gefunden im Internet: URL:https://pubs.acs.org/doi/pdf/10.1021/j a01130a043>
- CAVDAR H ET AL: "Ring opening of epoxides with NaHSO"4: isolation of @b-hydroxy sulfate esters and an effective synthesis for trans-diols", TETRAHEDRON, ELSEVIER SIENCE PUBLISHERS, AMSTERDAM, NL, Bd. 65, Nr. 5, 31. Januar 2009 (2009-01-31), Seiten 985-989, XP025816078, ISSN: 0040-4020, DOI: 10.1016/J.TET.2008.11.092 [gefunden am 2008-12-03]

## Beschreibung

Die vorliegende Erfindung betrifft spezielle Acetal-/Ketal-basierte Duftvorläuferverbindungen der Formel (III), die sich von Epoxiden und Duftstoffaldehyden und -ketonen ableiten, ein Verfahren zu deren Herstellung sowie Acetal-/Ketal-basierte Insektenabwehrmittelvorläuferverbindungen der Formel (VI), die sich von Epoxiden und Insektenabwehrmittelaldehyden und -ketonen ableiten und ein Verfahren zu deren Herstellung. Drüber hinaus betrifft die vorliegende Erfindung Wasch- und Reinigungsmittel, Luftpflegemittel und kosmetische Mittel, die die besagten Duftvorläuferverbindungen enthalten sowie Insektenabwehrmittel, die die besagten Insektenabwehrmittelvorläuferverbindungen enthalten. Des Weiteren betrifft die vorliegende Erfindung ein Verfahren zur langanhaltenden Beduftung von Oberflächen unter Verwendung der erfindungsgemäßen Duftvorläuferverbindungen und Mittel.

Wasch- oder Reinigungsmittel bzw. kosmetische Mittel enthalten zumeist Duftstoffe, die den Mitteln einen angenehmen Geruch verleihen. Die Duftstoffe können dabei auch dazu dienen, den Geruch anderer Inhaltstoffe zu maskieren, so dass beim Verbraucher ein angenehmer Geruchseindruck entsteht.

Insbesondere im Bereich Waschmittel sind Duftstoffe wichtige Bestandteile der Zusammensetzung, da die Wäsche sowohl im feuchten als auch im trockenen Zustand einen angenehmen und möglichst auch frischen Duft aufweisen soll. Die Verwendung von Duftstoffen ist grundsätzlich problematisch, da es sich bei diesen um mehr oder minder leicht flüchtige Verbindungen handelt, aber dennoch ein langanhaltender Dufteffekt angestrebt wird. Insbesondere bei denjenigen Riechstoffen, die die frischen und leichten Noten des Parfüms darstellen und infolge ihres hohen Dampfdrucks besonders flüchtig sind, ist die gewünschte Langlebigkeit des Dufteindrucks kaum erreichbar.

Eine verzögerte Duftfreisetzung kann z.B. durch sogenannte Duft(stoff)vorläuferverbindungen erfolgen. Diese Duftvorläuferverbindungen basieren auf Duftstoffverbindungen, beispielsweise Duftstoffaldehyden oder -ketonen, welche mit weiteren Verbindungen zu den genannten Duftvorläuferverbindungen umgesetzt werden, wobei diese dann in der Lage sind, die eigentliche Duftstoffverbindung verzögert wieder freizusetzen (beispielsweise durch Hydrolyse oder photochemisch). Obwohl sowohl Duftstoffaldehyde und -ketone als Ausgangsmaterialien für Duftvorläuferverbindungen beschrieben wurden, sind bis dato keine kommerziell geeigneten Duftvorläuferverbindungen mit Ketonen verfügbar. Die Aufgabe der vorliegenden Erfindung war deshalb die Bereitstellung von Duftvorläuferverbindungen, insbesondere auf Basis von Duftstoffketonen, welche durch eine einfache Synthese zu erhalten sind. Ferner sollen die Duftvorläuferverbindungen bevorzugt für den Einsatz in Konsumgütern, insbesondere Wasch- und Reinigungsmitteln geeignet sein. Wünschenswert ist hier ebenfalls, dass die Duftvorläuferverbindungen ferner eine bessere Haftung auf der Oberfläche auf die sie aufgebracht werden, bevorzugt einem Textil, erzielen.

Dieselben Effekten, also verzögerte und länger andauernde Freisetzung sind auch bei Insektenabwehrmitteln gewünscht, so dass es zu einer länger anhaltenden Wirkung vor insbesondere stechenden und saugenden Insekten kommt.

DE 10 2016 223412 A1 betrifft Duftspeicherstoffe. Insbesondere betrifft die besagte Druckschrift spezielle cyclische Ketale, die als Duftspeicherstoffe fungieren, sowie Wasch- und Reinigungsmittel, kosmetische Mittel sowie Luftpflegemittel, welche solche Ketale enthalten und Verfahren zur langanhaltenden Beduftung von Oberflächen sowie zur Abwehr von Insekten.

WO 97/34986 A1 betrifft Waschmittelzusammensetzungen, die Acetal- oder Ketal-Duftstoffvorläuferverbindungen enthalten, und Verfahren zur Beduftung von Textilartikeln und anderen Oberflächen mittels der besagten organischen Vorläuferverbindungen.

Die Erfinder der vorliegenden Erfindung haben gefunden, dass die Aufgabe durch die speziellen Duftvorläuferverbindungen der Formel (III), die sich von Epoxiden und Duftstoffaldehyden und -ketonen ableiten sowie die speziellen Insektenabwehrmittelvorläuferverbindungen der Formel (VI), die sich von Epoxiden und Insektenabwehrmittelaldehyden und -ketonen ableiten, gelöst werden kann.

Die vorliegende Erfindung und ihre bevorzugten Ausführungsformen sind aus den anhängenden Ansprüchen ersichtlich.

Diese und weitere Ausführungsformen, Merkmale und Vorteile der Erfindung werden für den Fachmann aus dem Studium der folgenden detaillierten Beschreibung und Ansprüche ersichtlich. Es ist selbstverständlich, dass die hierin enthaltenen Beispiele die Erfindung beschreiben und veranschaulichen sollen, diese aber nicht einschränken und insbesondere die Erfindung nicht auf die Beispiele beschränkt ist.

"Mindestens ein", wie hierin verwendet, bezieht sich auf 1 oder mehr, beispielsweise 2, 3, 4, 5, 6, 7, 8, 9 oder mehr. Im Zusammenhang mit den hierin beschriebenen Duftvorläuferverbindungen bezieht sich diese Angabe nicht auf die absolute Menge an Molekülen, sondern auf die Art der Verbindung. "Mindestens eine Duftvorläuferverbindung" bedeutet daher beispielsweise, dass nur eine Art von Duftvorläuferverbindung oder mehrere verschiedene Arten von Duftvorläuferverbindungen, ohne Angaben über die Menge der einzelnen Verbindungen zu machen, enthalten sein können.

Alle im Zusammenhang mit dem hierin beschriebenen Verfahren angegeben Mengenangaben beziehen sich, sofern nichts anderes angegeben ist, auf Gew.-% jeweils bezogen auf das Gesamtgewicht der Zusammensetzung. Des Weiteren beziehen sich derartige Mengenangaben, die sich auf mindestens einen Bestandteil beziehen, immer auf die Gesamtmenge dieser Art von Bestandteil, der in der Zusammensetzung enthalten ist, sofern nicht explizit etwas anderes angegeben ist. Das heißt, dass sich derartige Mengenangaben, beispielsweise im Zusammenhang mit "mindestens einer Duftvorläuferverbindung", auf die Gesamtmenge von Duftvorläuferverbindungen, welche in der Zusammensetzung enthalten sind, bezieht, wenn nicht explizit etwas anderes angegeben ist. Zahlenwerte, die hierin ohne Dezimalstellen angegeben sind, beziehen sich jeweils auf den vollen angegebenen Wert mit einer Dezimalstelle. So steht beispielsweise "99 %" für "99,0 %".

Numerische Bereiche, die in dem Format "in/von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Substituiert", wie hierin im Zusammenhang mit der Definition der Duftstoffvorläuferverbindungen der Formeln (I) bis (III) oder der Insektenabwehrmittelvorläuferverbindung der Formeln (IV) bis (VI) verwendet, bedeutet, dass ein Wasserstoffatom durch einen anderen Rest ersetzt ist. Geeignete Reste schließen ein, sind aber nicht beschränkt auf, lineare oder verzweigte Kohlenwasserstoffgruppen mit bis zu 22 Kohlenstoffatomen, bevorzugt Kohlenwasserstoffgruppen mit bis zu 10 Kohlenstoffatomen, einschließlich Alkyl, Alkenyl, Alkinyl; -OH, -CN, -NO₂, -C(O)H, -C(O)OR', -C(O)NR'R", -NR'-C(O)-R", -NR'R", wobei R' und R" lineares oder verzweigtes Alkyl mit bis zu 12 Kohlenstoffatomen, bevorzugt lineares oder verzweigtes Alkyl mit bis zu 6 Kohlenstoffatomen, sind.

Die Begriffe "Riechstoff" und "Duftstoff" sind im Rahmen dieser Erfindung synonym zu verwenden. Ein Riechstoff ist eine Verbindung, die einen charakteristischen Geruch aufweist und zur Erzielung eines spezifischen Duftprofils eines Parfümöls oder einer Zusammensetzung beiträgt. Zu Riechstoffen gehören auch solche Verbindungen, die das Duftprofil eines Parfümöls oder einer Zusammensetzung dahingehend verändern, dass der Duft eine gewisse Tiefe erhält, was der Fachmann üblicherweise als Komplexität eines Duftes kennt.

Die vorliegende Erfindung betrifft insbesondere Duftvorläuferverbindungen der Formel (III): 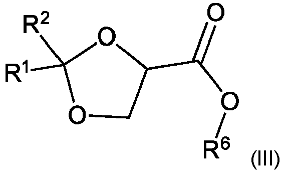 wobei
R¹ und R² unabhängig voneinander ausgewählt sind aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, oder R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann; und
R⁶ ausgewählt ist aus linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten;
mit der Maßgabe, dass sich der Rest R¹-C-R² von einem Duftstoffaldehyd der Formel R¹-C(=O)H oder einem Duftstoffketon der Formel R¹-C(=O)-R² ableitet;
wobei der Duftstoffaldehyd ausgewählt wird aus Adoxal (2,6,10-Trimethyl-9-undecenal), Cymal oder Cyclamenaldehyd (3-(4-Isopropylphenyl)-2-methylpropanal), Nympheal (3-(4-Isobutyl-2-methylphenyl)propanal), Ethylvanillin, Florhydral (3-(3-Isopropylphenyl)butanal]), Trifernal (3-Phenylbutyraldehyd), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), Triplal (2,4-Dimethyl-3-cyclohexen-1-carboxaldehyd), 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, Tetrahydrocitral (3,7-Dimethyloctanal), Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methyldecanal, 1-Nonanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Floral (4,8-Dimethyl-4,9-decadienal), Aldehyd C12MNA (2-Methylundecanal), Liminal (beta-4-Dimethylcyclohex-3-ene-1-propan-1-al), Methylnonylacetaldehyd, Hexanal, trans-2-Hexenal und Mischungen davon;
   und
wobei das Duftstoffketon ausgewählt wird aus Methyl-beta-naphthylketon, Moschusindanon (1,2,3,5,6,7-Hexahydro-1,1,2,3,3-pentamethyl-4H-inden-4-on), Calone (Methylbenzodioxepinon), Tonalid (6-Acetyl-1,1,2,4,4,7-hexamethyltetralin), alpha-Damascone, beta-Damascone, delta-Damascone, iso-Damascone, Damascenone, Methyldihydrojasmonat (Hedion), Menthon, Carvon, Kampfer, Koavon (3,4,5,6,6-Pentamethylhept-3-en-2-on), Fenchon, alpha-lonon, beta-lonon, Dihydro-beta-lonon, gamma-Methyl-lonon, Fleuramon (2-Heptylcyclopentanon), Frambinonmethylether (4-(4-Methoxyphenyl)butan-2-on), Dihydrojasmon, cis-Jasmon, 1-(1,2,3,4,5,6,7,8-Octahydro-2,3,8,8-tetramethyl-2-naphthalenyl)-ethan-1-on und Isomere davon, Methylcedrenylketon, Methylacetophenon, para-Methoxyacetophenon, Methyl-beta-naphtylketon, Benzylaceton, para-Hydroxyphenylbutanon, Sellerie-Keton(3-Methyl-5-propyl-2-cyclohexenon), 6-Isopropyldeca-hydro-2-naphton, Dimethyloctenon, Frescomenthe (2-Butan-2-ylcyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl (4-(1,3-Benzodioxol-5-yl)butan-2-on), Hexalon (1-(2,6,6-Trimethyl-2-cyclohexen-1-yl)-1,6-heptadien-3-on), Isocyclemon E (2-Acetonaphthon-1 ,2,3,4,5,6,7,8-octahydro-2,3,8,8-tetramethyl), Methylnonylketon, Methylcyclocitron, Methyllavendelketon, Orivon (4-tert-Amylcyclohexanon), 4-tert-Butylcyclohexanon, Delphon (2-Pentylcyclopentanon), Muscon (CAS 541 -91 -3), Neobutenon (1-(5,5-dimethyl-1-cyclohexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on, Tetrameran (6,10-dimethylundecen-2-on) und Mischungen davon.

Die Verbindungen der Formel (III) können in R oder S Konfiguration vorliegen, beispielsweise in S Konfiguration.

Die vorliegende Erfindung betrifft auch Insektenabwehrmittelvorläuferverbindungen der Formel (VI) 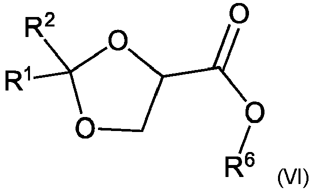 wobei
R¹ und R² unabhängig voneinander ausgewählt sind aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, oder R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann; und
R⁶ ausgewählt ist aus einem verzweigten oder linearen Alkylrest mit bis zu 12 Kohlenstoffatomen, besonders bevorzugt Methyl, Ethyl oder Propyl;
mit der Maßgabe, dass sich der Rest R¹-C-R² von einem Insektenabwehrmittelaldehyd der Formel R¹-C(=O)H oder einem Insektenabwehrmittelketon der Formel R¹-C(=O)-R² ableitet;
wobei das Insektenabwehrmittelaldehyd Callicarpenal ist,
wobei das Insektenabwehrmittelketon ein Keton ist ausgewählt aus der Gruppe bestehend aus 2-Undecanon, Allethrinen, Terpen-ketonen, Nootkaton und Mischungen davon.

Die Verbindungen der Formel (VI) können in R oder S Konfiguration vorliegen, beispielsweise in S Konfiguration.

Der Ausdruck "Rest R¹-C-R²" bezieht sich auf den entsprechenden Teil der Verbindung der Formeln (I) bis (VI), wobei dieser über das zentrale C-Atom als Bestandteil des Acetal-/Ketalrings an den Rest der Verbindung gebunden ist. Dieser Rest leitet sich von Duftstoffketonen oder -aldehyden bzw. Insektenabwehrmittelketonen oder -aldehyden ab, wobei das Sauerstoffatom des Aldehyds oder Ketons in dem gebildeten Acetal/Ketal an das zentrale C-Atom sowie den Rest des Acetal/Ketalrings, welcher durch Reaktion mit dem Epoxid entstanden ist gebunden ist. Dabei bindet das Sauerstoffatom des Epoxids bei der Reaktion an das zentrale C-Atom unter Ringschluss.

Aus den beschriebenen Duftvorläuferverbindungen der Formel (III) kann der Duftstoff durch Hydrolyse freigesetzt werden, insbesondere bei sauren pH-Werten, d.h. pH-Werten <7, beispielsweise <6 oder <5.

Aus den beschriebenen Insektenabwehrmittelvorläuferverbindungen der Formel (VI) kann das Insektenabwehrmittel durch Hydrolyse freigesetzt werden, insbesondere bei sauren pH-Werten, d.h. pH-Werten <7, beispielsweise <6 oder <5.

Die Duft- und Insektenabwehrmittelvorläuferverbindungen der vorliegenden Erfindung werden durch Umsetzen eines entsprechenden Epoxids mit dem Duftstoff bzw. Insektenabwehrmittel, hier ein Duftstoffaldehyd oder -keton bzw. Insektenabwehrmittelaldehyd oder -keton, insbesondere ein Duftstoffketon bzw. Insektenabwehrmittelketon, erhalten.

Gemäß der Erfindung ist das Duftstoffaldehyd ausgewählt werden aus Adoxal (2,6,10-Trimethyl-9-undecenal), Cymal oder Cyclamenaldehyd (3-(4-Isopropylphenyl)-2-methylpropanal), Nympheal (3-(4-Isobutyl-2-methylphenyl)propanal), Ethylvanillin, Florhydral (3-(3-Isopropylphenyl)butanal]), Trifernal (3-Phenylbutyraldehyd), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), Triplal (2,4-Dimethyl-3-cyclohexen-1-carboxaldehyd), 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, Tetrahydrocitral (3,7-Dimethyloctanal), Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methyldecanal, 1-Nonanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Floral (4,8-Dimethyl-4,9-decadienal), Aldehyd C12MNA (2-Methylundecanal), Liminal (beta-4-Dimethylcyclohex-3-ene-1-propan-1-al), Methylnonylacetaldehyd, Hexanal, trans-2-Hexenal und Mischungen davon.

Gemäß der Erfindung ist das Duftstoffketon ausgewählt aus Methyl-beta-naphthylketon, Moschusindanon (1,2,3,5,6,7-Hexahydro-1,1,2,3,3-pentamethyl-4H-inden-4-on), Calone (Methylbenzodioxepinon), Tonalid (6-Acetyl-1,1,2,4,4,7-hexamethyltetralin), alpha-Damascone, beta-Damascone, delta-Damascone, iso-Damascone, Damascenone, Methyldihydrojasmonat (Hedion), Menthon, Carvon, Kampfer, Koavon (3,4,5,6,6-Pentamethylhept-3-en-2-on), Fenchon, alpha-lonon, beta-Ionon, Dihydro-beta-lonon, gamma-Methyl-lonon, Fleuramon (2-Heptylcyclopentanon), Frambinonmethylether (4-(4-Methoxyphenyl)butan-2-on), Dihydrojasmon, cis-Jasmon, 1-(1,2,3,4,5,6,7,8-Octahydro-2,3,8,8-tetramethyl-2-naphthalenyl)-ethan-1-on und Isomere davon, Methylcedrenylketon, Methylacetophenon, para-Methoxyacetophenon, Methyl-beta-naphtylketon, Benzylaceton, para-Hydroxyphenylbutanon, Sellerie-Keton(3-Methyl-5-propyl-2-cyclohexenon), 6-Isopropyldeca-hydro-2-naphton, Dimethyloctenon, Frescomenthe (2-Butan-2-ylcyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)in-danon, 4-Damascol, Dulcinyl (4-(1,3-Benzodioxol-5-yl)butan-2-on), Hexalon (1-(2,6,6-Trimethyl-2-cyclohexen-1-yl)-1,6-heptadien-3-on), Isocyclemon E (2-Acetonaphthon-1 ,2,3,4,5,6,7,8-octahydro-2,3,8,8-tetramethyl), Methylnonylketon, Methylcyclocitron, Methyllavendelketon, Orivon (4-tert-Amylcyclohexanon), 4-tert-Butylcyclohexanon, Delphon (2-Pentylcyclopentanon), Muscon (CAS 541 -91 -3), Neobutenon (1-(5,5-dimethyl-1-cyclo-hexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on, Tetrameran (6,10-dimethylundecen-2-on) und Mischungen davon.

Bevorzugt ist das Duftstoffaldehyd oder -keton ausgewählt aus Dihydro-beta-ionon, Frambinonmethylether, Hedion, Benzylaceton, Calone, Decanal, Cyclamenaldehyd und Melonal, insbesondere aus Dihydro-beta-ionon, Frambinonmethylether, Hedion und Melonal.

Duftstoffaldehyde und/oder Duftstoffketone sind dem Fachmann bekannt und auch in der Patentliteratur, beispielsweise in US 2003/0158079 A1, Absätze [0154] und [0155] beschrieben. Für weitere geeignete Duftstoffe sei auf Steffen Arctander, Aroma Chemicals Band 1 und Band 2 (veröffentlicht 1960 bzw. 1969, Neuauflage 2000; ISBN: 0-931710-37-5 und 0-931710-38-3) verwiesen.

Die von den Duftstoffketonen bzw. -aldehyden abgeleiteten Reste in den Verbindungen der Formel (III) sind daher die Reste der vorstehend genannten Verbindungen. Ein von Decanal abgeleiteter Rest ist daher beispielsweise der Rest H₃C(CH₂)₈(C)H, wobei das (C) über zwei Sauerstoffatome im Acetal-/Ketalring gebunden ist.

Das Insektenabwehrmittelaldehyd ist Callicarpenal.

Das Insektenabwehrmittelketon ist ein Keton ausgewählt aus der Gruppe bestehend aus 2-Undecanon, Allethrinen, Terpen-ketonen, Nootkaton und Mischungen davon.

Ein Verfahren zur Herstellung der Duftvorläuferverbindungen der Formel (III) bzw. Insektenabwehrmittelvorläuferverbindungen der Formel (VI) kann das Umsetzen einer Verbindung der allgemeinen Formel (VII) umfassen: 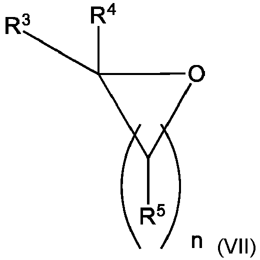 wobei
R³ H ist,
R⁴ ein Esterrest der Formel -C(=O)-O-R⁶ ist,
R⁵ H ist,
n 1 entspricht, und
R⁶ wie in Formel (III) oder Formel (VI) definiert ist,
mit Aldehyden der Formel R¹-C(=O)H oder Ketonen der Formel R¹-C(=O)-R² oder Mischungen davon, wobei R¹ und R² wie in Formel (III) oder Formel (VI) definiert sind.

In einer bevorzugten Ausführungsform ist die Verbindung (VII) eine Verbindung der allgemeinen Formel (VIII) 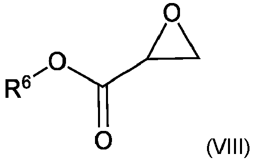 wobei R⁶ ausgewählt ist aus linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, bevorzugt aus verzweigten oder linearen gesättigten Kohlenwasserstoffresten mit bis zu 20 Kohlenstoffatomen, insbesondere einem verzweigten oder linearen Alkylrest mit bis zu 12 Kohlenstoffatomen, besonders bevorzugt Methyl, Ethyl oder Propyl, insbesondere Methyl.

Die Verbindungen der Formel (VIII) können in R oder S Konfiguration vorliegen, beispielsweise in S Konfiguration. Die Verbindung der Formel (VIII) kann insbesondere Methyl-(2S)-glycinat sein.

Die Umsetzung findet dabei bevorzugt unter Stickstoffatmosphäre statt. Ferner wird die Umsetzung bevorzugt in einem geeigneten Lösungsmittel durchgeführt. Ein geeignetes Lösungsmittel ist beispielsweise Dichlormethan. Bevorzugt wird dabei ein Katalysator verwendet, stärker bevorzugt ein Übergangsmetallhalogenid, insbesondere Zinn(VI)chlorid. Dieses wird bevorzugt zu der Mischung, welches die Edukte und das Lösungsmittel enthält zugetropft, insbesondere bei Temperaturen von -30 bis 0 °C. Das so erhaltene Gemisch mit Katalysator wird dann bevorzugt für mehrere Stunden, bevorzugt 2 bis 60, insbesondere 12 bis 48, bei Raumtemperatur gerührt. Danach kann bevorzugt eine Erwärmung erfolgen, insbesondere unter Rückfluss. Das erhaltene Umsetzungsprodukt wird nach üblichen Verfahren isoliert und gegebenenfalls gereinigt.

Die vorliegende Erfindung betrifft ferner Wasch-, Reinigungs-, Luftpflege- oder kosmetische Mittel, welche mindestens eine der Duftvorläuferverbindungen der vorliegenden Erfindung enthalten. In bevorzugten Ausführungsformen ist die mindestens eine Duftvorläuferverbindung in einer Gesamtmenge von 0,001 bis 5 Gew.-%, vorteilhafterweise von 0,005 bis 4 Gew.-%, weiter vorteilhaft von 0,01 bis 2 Gew.-%, jeweils bezogen auf das Gesamtgewicht des jeweiligen Mittels, enthalten.

Ebenso betrifft die vorliegende Erfindung ein Insektenabwehrmittel, welches mindestens eine Insektenabwehrmittelvorläuferverbindung der vorliegenden Erfindung enthält. In bevorzugten Ausführungsformen ist die Insektenabwehrmittelvorläuferverbindung in 0,001 bis 95 Gew.-%, bevorzugt 1 bis 50 Gew.-%, stärker bevorzugt 10 bis 20 Gew.-% enthalten, basierend auf dem Gesamtgewicht des Insektenabwehrmittels.

Die oben beschriebenen Duftvorläuferverbindungen der Formel (III) können in den Mitteln des erfindungsgemäßen Verfahrens als Mischungen mit mindestens einem weiteren Duftstoff oder mindestens einer weiteren Duftvorläuferverbindung, welche von der Duftvorläuferverbindung nach Formel (I) verschieden ist, eingesetzt werden. In den Duftvorläuferverbindungen der Formel (I) 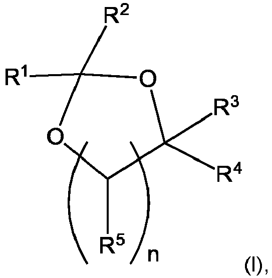
sind R¹ und R² unabhängig voneinander ausgewählt sind aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, oder R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
ist R³ ausgewählt aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, bevorzugt aus H oder verzweigten oder linearen gesättigten Kohlenwasserstoffresten mit bis zu 20 Kohlenstoffatomen, insbesondere einem verzweigten oder linearen Alkylrest mit bis zu 12 Kohlenstoffatomen, besonders bevorzugt H;
ist R⁴ ein Esterrest, Ketonrest, Aldehydrest, -CN, -NO₂, -Br, -CI oder -F, bevorzugt ein Esterrest, -CI oder -F, stärker bevorzugt ein Esterrest, insbesondere -C(=O)OC₁₋₁₂Alkyl;
ist R⁵ ausgewählt aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, bevorzugt aus H oder verzweigten oder linearen gesättigten Kohlenwasserstoffresten mit bis zu 20 Kohlenstoffatomen, insbesondere einem verzweigten oder linearen Alkylrest mit bis zu 12 Kohlenstoffatomen, besonders bevorzugt H; und
ist n eine ganze Zahl von 1 bis 3, bevorzugt 1 oder 2, stärker bevorzugt 1;
mit der Maßgabe, dass sich der Rest R¹-C-R² von einem Duftstoffaldehyd der Formel R¹-C(=O)H oder einem Duftstoffketon der Formel R¹-C(=O)-R² ableitet, bevorzugt von einem Duftstoffketon der Formel R¹-C(=O)-R² .

Die weiteren Duftstoffe, die in den Zusammensetzungen optional enthalten sein können, sind keinen besonderen Beschränkungen unterworfen. So können einzelne Duftstoffverbindungen natürlichen oder synthetischen Ursprungs, z.B. vom Typ der Ester, Ether, Aldehyde, Ketone, Alkohole und Kohlenwasserstoffe, verwendet werden. Duftstoffverbindungen vom Typ der Ester sind z.B. Benzylacetat, Phenoxyethylisobutyrat, *p*-*tert*-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden die oben genannten z.B. die linearen Alkanale mit 8 bis 18 C-Atomen, Citral, Citronellal, Citronellyloxyacetaldehyd, Cyclamenaldehyd (3-(4-propan-2-ylphenyl)butanal), Lilial und Bourgeonal, zu den Ketonen z.B. die lonone, [alpha]-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Duftstoffe verwendet, die gemeinsam eine ansprechende Duftnote erzeugen.

Die Zusammensetzungen können ferner auch natürliche Duftstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl. Weitere herkömmliche Duftstoffe, die im Rahmen der vorliegenden Erfindung in den erfindungsgemäßen Mitteln enthalten sein können, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Copaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wntergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iran, Isoeugenol, Isoeugenolmethyl-ether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, n-Nonylaldehyd, Nonylalkohol, n-Octyl-aldehyd, p-Oxy-Acetophenon, Pentadekanolid, β-Phenylethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanillin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Die oben beschriebenen Insektenabwehrmittelvorläuferverbindungen der Formel (VI) können in den erfindungsgemäßen Mitteln als Mischungen mit mindestens einem weiteren Insektenabwehrmittel oder mindestens einer weiteren Insektenabwehrmittelvorläuferverbindung, welche von der Insektenabwehrmittelvorläuferverbindung nach Formel (IV) verschieden ist, eingesetzt werden. In den Insektenabwehrmittelvorläuferverbindungen der Formel (IV) 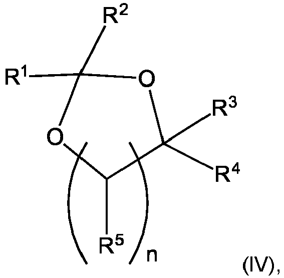
sind R¹ und R² unabhängig voneinander ausgewählt sind aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, oder R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann;
ist R³ ausgewählt aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, bevorzugt aus H oder verzweigten oder linearen gesättigten Kohlenwasserstoffresten mit bis zu 20 Kohlenstoffatomen, insbesondere einem verzweigten oder linearen Alkylrest mit bis zu 12 Kohlenstoffatomen, besonders bevorzugt H;
ist R⁴ ein Esterrest, Ketonrest, Aldehydrest, -CN, -NO₂, -Br, -CI oder -F, bevorzugt ein Esterrest, -CI oder -F, stärker bevorzugt ein Esterrest, insbesondere -C(=O)OC₁₋₁₂Alkyl;
ist R⁵ ausgewählt aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, bevorzugt aus H oder verzweigten oder linearen gesättigten Kohlenwasserstoffresten mit bis zu 20 Kohlenstoffatomen, insbesondere einem verzweigten oder linearen Alkylrest mit bis zu 12 Kohlenstoffatomen, besonders bevorzugt H; und
ist n eine ganze Zahl von 1 bis 3, bevorzugt 1 oder 2, stärker bevorzugt 1;
mit der Maßgabe, dass sich der Rest R¹-C-R² von einem Insektenabwehrmittelaldehyd der Formel R¹-C(=O)H oder einem Insektenabwehrmittelketon der Formel R¹-C(=O)-R² ableitet, bevorzugt von einem Insektenabwehrmittelketon der Formel R¹-C(=O)-R².

Es ist außerdem möglich, dass die mindestens eine Duftvorläuferverbindung der Formel (III) bzw. die mindestens eine Insektenabwehrmittelvorläuferverbindung der Formel (VI) mit den korrespondierenden Aldehyden und/oder Ketonen eingesetzt werden. Gemäß einer bevorzugten Ausführungsform zeichnen sich derartige Zusammensetzungen dadurch aus, dass das Molverhältnis von Duftstoffaldehyd und/oder -Keton bzw. Insektenabwehrmittelaldehyd und/oder -keton zu der korrespondierenden Vorläuferverbindung der Formel (III) bzw. (VI) 20:1 bis 1:20, bevorzugt 10:1 bis 1:10, stärker bevorzugt 5:1 bis 1:5, noch stärker bevorzugt 3:1 bis 1:3, noch stärker bevorzugt 2:1 bis 1:2 und insbesondere 1,2:1 bis 1:1,2 beträgt.

Die im Verfahren einzusetzenden Wasch- und Reinigungsmittel können ferner anionische, nichtionische, kationische, amphotere oder zwitterionische Tenside oder Mischungen davon enthalten. Ferner können diese Mittel in fester oder flüssiger Form vorliegen.

Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar. Geeignete anionische Tenside sind insbesondere Seifen und solche, die Sulfat- oder Sulfonat-Gruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen. Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X- ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X- für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden z.B. mit Dimethylsulfat quaterniert. In Frage kommende QAV sind beispielweise Benzalkoniumchlorid (N-Alkyl-N,N-dimethylbenzylammoniumchlorid), Benzalkon B (m,p-Dichlorbenzyldimethyl-C₁₂-alkylammoniumchlorid, Benzoxoniumchlorid (Benzyldodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzyl-ammoniumchlorid), Dialkyldimethylammoniumchloride wie Di-n-decyl-dimethyl-ammoniumchlorid, Didecyldimethylammoniumbromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkylbenzyl-dimethylammoniumchlorid.

Bevorzugte Esterquats sind Methyl-N-(2-hydroxyethyl)-N,N-di(talgacyl-oxyethyl)ammonium-metho-sulfat, Bis-(palmitoyl)-ethyl-hydroxyethyl-methyl-ammonium-methosulfat oder Methyl-N,N-bis(acyl-oxyethyl)-N-(2-hydroxyethyl)ammonium-methosulfat. Handelsübliche Beispiele sind die von der Firma Stepan unter dem Warenzeichen Stepantex^{®} vertriebenen Methylhydroxyalkyldialkoyloxyalkylammoniummethosulfate oder die unter dem Handelsnamen Dehyquart^{®} bekannten Produkte der Firma BASF SE beziehungsweise die unter der Bezeichnung Rewoquat^{®} bekannten Produkte des Herstellers Evonik.

Ferner können die Wasch- und Reinigungsmittel weitere Inhaltsstoffe enthalten, die die anwendungstechnischen und/oder ästhetischen Eigenschaften der Zusammensetzung abhängig von dem beabsichtigten Verwendungszweck weiter verbessern. Im Rahmen der vorliegenden Erfindung können sie Builder, Bleichmittel, Bleichaktivatoren, Bleichkatalysatoren, Esterquats, Silikonöle, Emulgatoren, Verdicker, Elektrolyte, pH-Stellmittel, Fluoreszenzmittel, Farbstoffe, Hydrotope, Schauminhibitoren, Antiredepositionsmittel, Lösungsmittel, Enzyme, optischen Aufheller, Vergrauungsinhibitoren, Einlaufverhinderer, Knitterschutzmittel, Farbübertragungsinhibitoren, Farbschutzmittel, Benetzungsverbesserer, antimikrobiellen Wirkstoffen, Germizide, Fungizide, Antioxidantien, Korrosionsinhibitoren, Klarspüler, Konservierungsmittel, Antistatika, Bügelhilfsmittel, Phobier- und Imprägniermittel, Perlglanzmittel, Polymere, Quell- und Schiebefestmittel sowie UV-Absorber enthalten, ohne darauf beschränkt zu sein.

Die Mengen der einzelnen Inhaltsstoffe in den Wasch- und Reinigungsmitteln orientieren sich jeweils am Einsatzzweck der betreffenden Zusammensetzung und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der Zusammensetzungen wird man beispielsweise den Tensidgehalt höher oder niedriger wählen. Üblicherweise kann z.B. der Tensidgehalt beispielsweise von Waschmitteln von 10 bis 50 Gew.-%, bevorzugt von 12,5 bis 30 Gew.-% und stärker bevorzugt von 15 bis 25 Gew.-% betragen.

Die Wasch- und Reinigungsmittel können beispielsweise mindestens einen wasserlöslichen und/oder wasserunlöslichen, organischen und/oder anorganischen Builder enthalten. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Die organischen Buildersubstanzen können, insbesondere zur Herstellung flüssiger Wasch- und Reinigungsmittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Organische Buildersubstanzen können, falls gewünscht, in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, falls gewünscht, in Mengen von bis zu 50 Gew.-%, vorzugsweise nicht über 40 Gew.-% und in flüssigen Zusammensetzungen insbesondere von 1 Gew.-% bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in Wasch- oder Reinigungsmitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2.8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁·yH₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl beta- als auch delta-Natriumdisilikate (Na₂Si₂O₅·yH₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können eingesetzt werden. In einer weiteren bevorzugten Ausführungsform wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform der Textilbehandlungs- oder Reinigungsmittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Zusammensetzungen, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind, falls gewünscht, bevorzugt in Mengen bis zu 60 Gew.-%, insbesondere von 5 Gew.-% bis 40 Gew.-%, enthalten. Wäschenachbehandlungsmittel, wie z.B. Weichspüler, sind bevorzugt frei von anorganischem Builder.

Geeignete Inhaltsstoffe und Rahmenzusammensetzungen für Wasch- und Reinigungsmittelzusammensetzungen (beispielsweise für Waschmittel und Weichspüler) sind beispielsweise in der EP 3 110 393 B1 offenbart.

Geeignete Inhaltsstoffe und Rahmenzusammensetzungen für kosmetische Mittel, wie Haarpflegemittelzusammensetzungen, sind beispielsweise in der DE 102017215071 A1 offenbart.

### Beispiele

### Allgemeine Arbeitsvorschrift Umsetzung Epoxide und Ketone:

Das gewünschte Produkt wurde unter Schlenk-Bedingungen mit Stickstoff als Inertgas synthetisiert. Hierfür wurden zunächst 2,0 g Molsieb (0,3 nm) unter Vakuum in einem 50 mL Zweihalskolben ausgeheizt und so aktiviert. Anschließend wurden 30 mL Dichlormethan (99,8% extra dry) vorgelegt und 20 mmol Methyl-(2S)-glycidat und 10 mmol Keton hinzugegeben. Es wurde 30 Minuten bei Raumtemperatur gerührt und die Mischung anschließend mittels einer Eis-/Kochsalzmischung auf -20 °C gekühlt. Bei dieser Temperatur wurden 10 mmol Zinn(IV)chlorid tropfenweise hinzugegeben. Nach einer Stunde wurde das Reaktionsgemisch auf Raumtemperatur erwärmt. Unter regelmäßiger DC und GC Kontrolle wurde das Reaktionsgemisch 48 Stunden bei Raumtemperatur gerührt und anschließend für eine Stunde am Rückfluss erhitzt. Nach dem Abkühlen erfolgte die Aufarbeitung. Dazu wurde die Mischung zunächst mit 50 ml einer gesättigten NaCl-Lösung versetzt, um die Reaktion abzubrechen. Die Phasen wurden getrennt und die wässrige Phase dreimal mit je 10 mL Dichlormethan extrahiert, die organischen Phasen vereinigt, über Natriumsulfat getrocknet und filtriert. Das Dichlormethan wurde im Vakuum abdestilliert und anschließend das Roh-Produkt mithilfe einer CombiFlash^{®} Rf über eine 40 g Silica Flash Column (RediSep^{®}Rf) durch ein n-Hexan/Ethylacetat-Gemisch als Laufmittel aufgereinigt.

### Beispiel 1: Umsetzung mit Frambinonmethylether nach allgemeiner Arbeitsvorschrift Es wurden 7,1 mmol des gewünschten Produktes als gelbliches Öl erhalten.

Rf = 0,27 (n-Hex:EE; 7:1). FTIR (film): ṽ = 2954, 1761, 1736, 1612, 1584, 1512, 1439, 1378, 1299, 1243, 1200, 1178, 1109, 1065, 1035, 955, 867, 844, 821, 753, 637, 515 cm⁻¹. ¹H-NMR (400 MHz, CDCl₃): δ = 7,13 - 7,01 (m, 2H), 6,90 - 6,80 (m, 2H), 4,69 - 4,59 (m, 1H), 4,15 - 4,09 (m, 1H), 4,35 - 4.,25 (m, 1H), 3,79 (s, 3H), 3,77 (s, 3H), 2,72 - 2,63 (m, 2H), 2,04 - 1,93 (m, 2H), 1,48 (s, 3H)*, 1,40 (s, 3H)*. ¹³C-NMR (125,8 MHz, CDCl₃): δ = 172,1 (C_{quart})*, 171,7 (C_{quart})*, 158,1 (C_{quart}), 134,4 (C_{quart}), 129,6 (CH, 2C), 114,2 (CH, 2C), 113,1 (C_{quart}), 74,6 (CH)*, 74,4 (CH)*, 67,8 (CH₂)*, 66,2 (CH₂)*, 55,6 (CH₃), 52,8 (CH₃)*, 52,7 (CH₃)*, 41,5 (CH₂), 29,7 (CH₂), 24,5 (CH₃)*, 24,3 (CH₃)* ppm. MS (ESI, positive ion): 281 ([M+H]⁺, 100%).
*Es liegt ein Diastereomeren-Gemisch in einem Verhältnis von 1:1 vor.

### Beispiel 2: Umsetzung mit Benzylaceton nach allgemeiner Arbeitsvorschrift. Es wurden 4,2 mmol des gewünschten Produktes als gelbliches Öl erhalten.

R_{f} = 0,29 (Hexan:EE; 7:1). FTIR (film): ṽ = 2954, 1759, 1437, 1201, 1069, 865, 749, 699, 524 cm⁻¹. ¹H-NMR (400 MHz, CDCl₃): 7,30 - 7,15 (m, 5H), 4,67 - 4,60 (m, 1H), 4,32 - 4,27 (m, 1H), 4,15 - 4,10 (m, 1H), 3,80 (s, 3H), 2,80 - 2,71 (m, 2H), 2,09 - 1,96 (m, 2H), 1,51 (s, 3H)*, 1,42 (s, 3H)* ppm. ¹³C-NMR (125,8 MHz, CDCl₃): δ = 172,1 (C_{quart})*, 171,7 (C_{quart})*, 142,2 (C_{quart}), 128,8 (CH, 2C), 128,4 (CH, 2C), 126,4 (CH), 113,0 (C_{quart}), 74,6 (CH)*, 74,4 (CH)*, 67,8 (CH₂), 52,8 (CH₃), 41,2 (CH₂), 30,6 (CH₂), 24,0 (CH₃)*, 23,8 (CH₃)* ppm. MS (ESI, positive ion): 251 ([M+H]⁺, 100%).
*Es liegt ein Diastereomeren-Gemisch in einem Verhältnis von 1:1 vor.

### Beispiel 3: Umsetzung mit β-Dihydroionon nach allgemeiner Arbeitsvorschrift. Es wurden 4,6 mmol des gewünschten Produktes als gelbliches Öl erhalten.

Rf = 0,36 (Hexan:EE; 7:1). FTIR (film): ṽ = 2930, 1763, 1377, 1203, 1068, 855, 736, 404 cm⁻¹. ¹H-NMR (400 MHz, CDCl₃): δ = 4,61 - 4,55 (m, 1H), 4,26 - 4,22 (m, 1H), 4,09 - 4,05 (m, 1H), 3,76 (s, 3H)*, 3,74 (s, 3H)*, 2,13 - 2,04 (m, 2H), 1,88 - 1,85 (t, *J* = 6,2 Hz, 2H), 1,80 - 1,73 (m, 1H), 1,72 - 1,68 (m, 1H), 1,60 - 1,55 (m, 3H), 1,55 - 1,50 (m, 2H), 1,44 - 1,34 (m, 3H), 1,28 - 1,23 (m, 1H), 0,98 - 0,82 (m, 6H), 0,81 - 0,75 (m, 1H) ppm. ¹³C-NMR (125,8 MHz, CDCl₃): δ = 172,1 (C_{quart})*, 171,7 (C_{quart})*, 136,7 (C_{quart}), 127,7 (C_{quart}), 113,5 (C_{quart}), 74,6 (CH)*, 74,4 (CH)*, 67,8 (CH₂)*, 67,6 (CH₂)*, 53,8 (CH₂), 52,7 (CH₃), 40,2 (CH₂)*, 39,6 (CH₂)*, 35,2 (C_{quart}), 33,1 (CH₂), 28,8 (2C, CH₃), 24,1 (CH₃)*, 23,7 (CH₃)*, 23,3 (CH₂)*, 23,2 (CH₂)*, 20,0 (CH₃), 19,9 (CH₂) ppm. MS (ESI, positive ion): 297 ([M+H]⁺, 100%).
*Es liegt ein Diastereomeren-Gemisch in einem Verhältnis von 1:1 vor.

### Beispiel 4: Riechtest

Nachfolgende Werte in Tabelle 1 sind der Mittelwert aus den Bewertungen mindestens dreier Tester. Die Proben wurden wie folgt hergestellt: Es wurde eine ca. 200 mM Lösung der entsprechenden reinen Riechstoffe sowie deren Precursor in Ethanol hergestellt. Anschließend wurde die Lösung auf Papierteststreifen aufgetragen, indem diese in die vorgefertigte Lösung eingetaucht wurde. Nachdem die Lösungsmittelfront etwa 2-3 cm nach oben gestiegen war, wurde der Teststreifen zum Trocknen bei Raumtemperatur auf einem Labortablett abgelegt und nach der angegebenen Zeit direkt von mindestens zwei Personen abgerochen oder zuerst mit Wassernebel bzw. einer pH = 3 gepufferten Lösung (*) besprüht und anschließend von mindestens zwei Personen abgerochen. Skala: 0: Kein Geruch wahrnehmbar; 6: Sehr starker Duftreindruck.

**Tabelle 1: Riechtest Duftstoffe vs. Duftstoffvorläuferverbindungen (Precursor)**

| **Verbindung** | **15 min** | **6 h** | **6h H₂O*** | **6h pH3*** | **24 h** | **24 h H₂O*** | **24 h pH3*** | **48 h** | **48 h H₂O*** | **48 h pH3*** | **72 h** | **144 h** |
|---|---|---|---|---|---|---|---|---|---|---|---|---|
| β-Dihydroionon | 6,00 | | | | | | | 0,34 | | | 0,34 | |
| β-Dihydroionon-Precursor | 2,25 | | | | | | | 0,50 | | | 0,84 | |
| | | | | | | | | | | | | |
| Benzylaceton | 6,00 | | | | 2,00 | | | 1,00 | | | 0,84 | 0 |
| Benzylaceton-Precursor | 1,67 | | | | 2,50 | | | 2,50 | | | 1,84 | 1,00 |
| | | | | | | | | | | | | |
| FME | 2,67 | 2,50 | 2,17 | 2,34 | | 1,34 | 2,00 | | 1,67 | 1,50 | | 0,00 |
| FME-Precursor | 3,34 | 2,75 | 2,67 | 4,00 | | 3,17 | 4,00 | | 2,17 | 1,84 | | 0,30 |

| | | | | | | | | | | | | |
|---|---|---|---|---|---|---|---|---|---|---|---|---|
| FME = Frambinonmethylether | | | | | | | | | | | | |

## Patentansprüche

1. Duftvorläuferverbindungen der Formel (III) 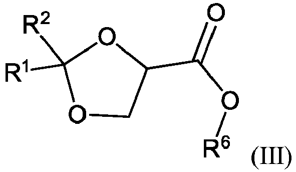 wobei
R¹ und R² unabhängig voneinander ausgewählt sind aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, oder R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann; und R⁶ ausgewählt ist aus linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten,
mit der Maßgabe, dass sich der Rest R¹-C-R² von einem Duftstoffaldehyd der Formel R¹-C(=O)H oder einem Duftstoffketon der Formel R¹-C(=O)-R² ableitet;
wobei der Duftstoffaldehyd ausgewählt wird aus Adoxal (2,6,10-Trimethyl-9-undecenal), Cymal oder Cyclamenaldehyd (3-(4-lsopropylphenyl)-2-methylpropanal), Nympheal (3-(4-Isobutyl-2-methylphenyl)propanal), Ethylvanillin, Florhydral (3-(3-Isopropylphenyl)butanal]), Trifernal (3-Phenylbutyraldehyd), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), Triplal (2,4-Dimethyl-3-cyclohexen-1-carboxaldehyd), 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Dimethyl-6-octen-1-al, [(3,7-Dimethyl-6-octenyl)oxy]acetaldehyd, 4-lsopropylbenzylaldehyd, 1,2,3,4,5,6,7,8-Octahydro-8,8-dimethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, Tetrahydrocitral (3,7-Dimethyloctanal), Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methyldecanal, 1-Nonanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Floral (4,8-Dimethyl-4,9-decadienal), Aldehyd C12MNA (2-Methylundecanal), Liminal (beta-4-Dimethylcyclohex-3-ene-1-propan-1-al), Methylnonylacetaldehyd, Hexanal, trans-2-Hexenal und Mischungen davon, und
wobei das Duftstoffketon ausgewählt wird aus Methyl-beta-naphthylketon, Moschusindanon (1,2,3,5,6,7-Hexahydro-1,1,2,3,3-pentamethyl-4H-inden-4-on), Calone (Methylbenzodioxepinon), Tonalid (6-Acetyl-1,1,2,4,4,7-hexamethyltetralin), alpha-Damascone, beta-Damascone, delta-Damascone, iso-Damascone, Damascenone, Methyldihydrojasmonat (Hedion), Menthon, Carvon, Kampfer, Koavon (3,4,5,6,6-Pentamethylhept-3-en-2-on), Fenchon, alpha-Ionon, beta-Ionon, Dihydro-beta-lonon, gamma-Methyl-lonon, Fleuramon (2-Heptylcyclopentanon), Frambinonmethylether (4-(4-Methoxyphenyl)butan-2-on), Dihydrojasmon, cis-Jasmon, 1-(1,2,3,4,5,6,7,8-Octahydro-2,3,8,8-tetramethyl-2-naphthalenyl)-ethan-1-on und Isomere davon, Methylcedrenylketon, Methylacetophenon, para-Methoxyacetophenon, Methyl-beta-naphtylketon, Benzylaceton, para-Hydroxyphenylbutanon, Sellerie-Keton(3-Methyl-5-propyl-2-cyclohexenon), 6-lsopropyldeca-hydro-2-naphton, Dimethyloctenon, Frescomenthe (2-Butan-2-ylcyclohexan-1-on), 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(SH)in-danon, 4-Damascol, Dulcinyl (4-(1,3-Benzodioxol-5-yl)butan-2-on), Hexalon (1-(2,6,6-Trimethyl-2-cyclohexen-1-yl)-1,6-heptadien-3-on), Isocyclemon E (2-Acetonaphthon-1 ,2,3,4,5,6,7,8-octahydro-2,3,8,8-tetramethyl), Methylnonylketon, Methylcyclocitron, Methyllavendelketon, Orivon (4-tert-Amylcyclohexanon), 4-tert-Butylcyclohexanon, Delphon (2-Pentylcyclopentanon), Muscon (CAS 541 -91 -3), Neobutenon (1-(5,5-dimethyl-1-cyclo-hexenyl)pent-4-en-1-on), Plicaton (CAS 41724-19-0), Velouton (2,2,5-trimethyl-5-pentylcyclopentan-1-on), 2,4,4,7-Tetramethyl-oct-6-en-3-on, Tetrameran (6,10-dimethylundecen-2-on) und Mischungen davon.

2. Duftvorläuferverbindungen nach Anspruch 1, wobei die Verbindungen durch die Umsetzung eines Epoxids, bevorzugt Methylglycidat, mit mindestens einem Duftstoffaldehyd oder -keton ausgewählt aus Dihydro-beta-ionon, Frambinonmethylether, Hedion, Benzylaceton, Calone, Decanal, Cyclamenaldehyd, Melonal oder Mischungen davon erhalten werden.

3. Insektenabwehrvorläuferverbindungen der Formel (VI) 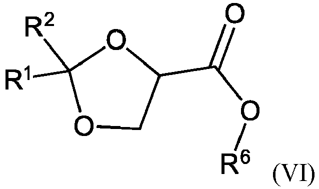 wobei
R¹ und R² unabhängig voneinander ausgewählt sind aus H oder linearen, verzweigten oder cyclischen, gesättigten oder ungesättigten, substituierten oder unsubstituierten Kohlenwasserstoffresten, die bis zu 20 Kohlenstoffatome und gegebenenfalls bis zu 6 Heteroatome enthalten, oder R¹ und R² gemeinsam mit dem Kohlenstoffatom an das sie gebunden sind einen cyclischen, gesättigten oder ungesättigten, Kohlenwasserstoffring mit bis zu 12 Kohlenstoffatomen bilden, der bis zu 6 Heteroatome enthalten kann; und
R⁶ ausgewählt ist aus einem verzweigten oder linearen Alkylrest mit bis zu 12 Kohlenstoffatomen, besonders bevorzugt Methyl, Ethyl oder Propyl;
mit der Maßgabe, dass sich der Rest R¹-C-R² von einem Insektenabwehrmittelaldehyd der Formel R¹-C(=O)H oder einem Insektenabwehrmittelketon der Formel R¹-C(=O)-R² ableitet;
wobei das Insektenabwehrmittelaldehyd Callicarpenal ist,
wobei das Insektenabwehrmittelketon ein Keton ist ausgewählt aus der Gruppe bestehend aus 2-Undecanon, Allethrinen, Terpen-ketonen, Nootkaton und Mischungen davon.

4. Wasch- oder Reinigungsmittel, enthaltend mindestens eine Duftvorläuferverbindung nach einem der Ansprüche 1 oder 2, wobei die Verbindung vorzugsweise in einer Gesamtmenge von 0,001 bis 5 Gew.-%, vorteilhafterweise von 0,005 bis 4 Gew.-%, weiter vorteilhaft von 0,01 bis 2 Gew.-%, jeweils bezogen auf das Gesamtgewicht des Mittels, enthalten ist.

5. Wasch- oder Reinigungsmittel nach Anspruch 4, **dadurch gekennzeichnet, dass**
(1) es mindestens ein Tensid ausgewählt aus anionischen, kationischen, nichtionischen, zwitterionischen, amphoteren Tensiden oder Mischungen daraus enthält,
und/oder
(2) es in fester oder flüssiger Form vorliegt.

6. Luftpflegemittel, enthaltend mindestens eine Duftvorläuferverbindung nach einem der Ansprüche 1 oder 2, wobei die Verbindung vorzugsweise in einer Gesamtmenge von 0,001 bis 5 Gew.-%, vorteilhafterweise von 0,005 bis 4 Gew.-%, weiter vorteilhaft von 0,01 bis 2 Gew.-%, bezogen auf das Gesamtgewicht des Mittels, enthalten ist.

7. Kosmetisches Mittel, enthaltend mindestens eine Duftvorläuferverbindung nach einem der Ansprüche 1 oder 2, wobei die Verbindung vorzugsweise in einer Gesamtmenge von 0,001 bis 5 Gew.-%, vorteilhafterweise von 0,005 bis 4 Gew.-%, weiter vorteilhaft von 0,01 bis 2 Gew.-%, bezogen auf das Gesamtgewicht des Mittels, enthalten ist.

8. Insektenabwehrmittel, enthaltend mindestens eine
Insektenabwehrmittelvorläuferverbindung nach Ansprüche 3, wobei die Verbindung vorzugsweise in einer Gesamtmenge von 1 bis 99 Gew.-%, bevorzugt 2 bis 50 Gew.-%, stärker bevorzugt 10 bis 20 Gew.-%, bezogen auf das Gesamtgewicht des Mittels, enthalten ist.

9. Verfahren zur Herstellung von Duftvorläuferverbindungen der Formel (III) wie in Anspruch 1 definiert, umfassend das Umsetzen
einer Verbindung nach der allgemeinen Formel (VII) 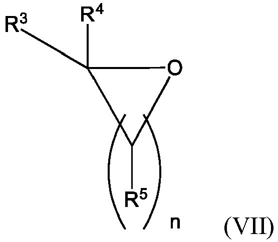
wobei
R³ H ist,
R⁴ ein Esterrest der Formel -C(=O)-O-R⁶ ist,
R⁵ H ist,
n 1 entspricht, und
R⁶ wie in Formel (III) von Anspruch 1 definiert ist,
mit Aldehyden der Formel R¹-C(=O)H oder Ketonen der Formel R¹-C(=O)-R² oder Mischungen davon, wobei R¹ und R² wie in Formel (III) definiert sind.

10. Verfahren zur Herstellung von Insektenabwehrmittelvorläuferverbindungen der Formel (VI) wie in Anspruch 3 definiert, umfassend das Umsetzen
einer Verbindung nach der allgemeinen Formel (VII) 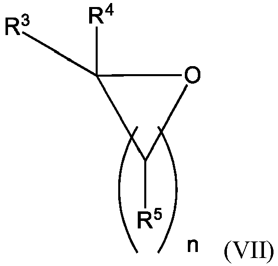 wobei
R³ H ist,
R⁴ ein Esterrest der Formel -C(=O)-O-R⁶ ist,
R⁵ H ist,
n 1 entspricht, und
R⁶ wie in Formel (VI) von Anspruch 3 definiert ist,
mit Aldehyden der Formel R¹-C(=O)H oder Ketonen der Formel R¹-C(=O)-R² oder Mischungen davon, wobei R¹ und R² wie in Formel (VI) definiert sind.

11. Verfahren zur langanhaltenden Beduftung von Oberflächen, **dadurch gekennzeichnet, dass** eine Duftvorläuferverbindung nach einem der Ansprüche 1 oder 2 oder ein Mittel nach Anspruch 4, 5 oder 7 auf die zu beduftende Oberfläche aufgebracht wird, wobei die Beduftung länger anhält, als wenn die jeweilige Duftstoffverbindung oder ein identisches Mittel in dem die Duftvorläuferverbindung durch die jeweilige Duftstoffverbindung ersetzt ist, eingesetzt würde.

## Claims

1. Fragrance precursor compounds of formula (III) 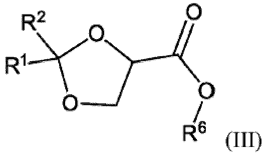 where
R¹ and R² are independently selected from H or linear, branched or cyclic, saturated or unsaturated, substituted or unsubstituted hydrocarbon groups, which contain up to 20 carbon atoms and optionally up to 6 heteroatoms, or R¹ and R² together with the carbon atom to which they are bonded form a cyclic, saturated or unsaturated, hydrocarbon ring with up to 12 carbon atoms, which may contain up to 6 heteroatoms; and R⁶ is selected from linear, branched or cyclic, saturated or unsaturated, substituted or unsubstituted hydrocarbon groups containing up to 20 carbon atoms and optionally up to 6 heteroatoms;
with the proviso that the group R¹-C-R² is derived from a fragrance aldehyde of formula R¹-C(=O)H or a fragrance ketone of formula R¹-C(=O)-R²;
wherein the fragrance aldehyde is selected from adoxal (2,6,10-trimethyl-9-undecenal), cymene or cyclamen aldehyde (3-(4-isopropylphenyl)-2-methylpropanal), nympheal (3-(4-isobutyl-2-methylphenyl)propanal) ethyl vanillin, Florhydral (3-(3-isopropylphenyl)butanal]), Trifernal (3-Phenylbutyraldehyde), helional (3-(3,4-methylendioxyphenyl)-2-methylpropanal), heliotropin, hydroxycitronellal, lauraldehyde, Lyral (3- and 4-(4-hydroxy-4-methylpentyl)-3-cyclohexene-1-carboxaldehyde), methyl nonyl acetaldehyde, Lilial (3-(4-tert-butylphenyl)-2-methylpropanal), phenylacetaldehyde, undecyl aldehyde, vanillin, 2,6,10-trimethyl-9-undecenal, 3-dodecen-1-al, alpha-n-amylcinnamaldehyde, melonal (2,6-dimethyl-5-heptenal), Triplal (2,4-dimethyl-3-cyclohexene-1-carboxaldehyde, 3-(4-tert-butylphenyl)-propanal, 2-methyl-3-(para-methoxyphenyl)propanal, 2-methyl-4-(2,6,6-timethyl-2(1)-cyclohexene-1-yl)butanal, 3-phenyl-2-propenal, cis-/trans-3,7-dimethyl-2,6-octadiene-1-al, 3,7-dimethyl-6-octen-1-al, [(3,7-dimethyl-6-octenyl)oxy]acetaldehyde, 4-isopropylbenzylaldehyde, 1,2,3,4,5,6,7,8-octahydro-8,8-dimethyl-2-naphthaldehyde, 2,4-dimethyl-3-cyclohexene-1-carboxaldehyde, 2-methyl-3-(isopropylphenyl)propanal, 1-decanal, 2,6-dimethyl-5-heptenal, 4-(tricyclo[5.2.1.0(2,6)]-decyliden-8)-butanal, octahydro-4,7-methane-1H-indencarboxaldehyde, 3-ethoxy-4-hydroxybenzaldehyde, para-ethyl-alpha, alpha-dimethyl hydrocinnamaldehyde, alpha-methyl-3,4-(methylenedioxy)-hydrocinnamaldehyde, 3,4-methylenedioxybenzaldehyde, alpha-n-hexylcinnamaldehyde, m-cymen-7-carboxaldehyde, alpha-methyl phenyl acetaldehyde, tetrahydrocitral (3,7-dimethyloctanal), undecenal, 2,4,6-trimethyl-3-cyclohexene-1-carboxaldehyde, 4-(3)(4-methyl-3-pentenyl)-3-cyclohexencarboxaldehyde, 1-dodecanal, 2,4-dimethylcyclohexene-3-carboxaldehyde, 4-(4-hydroxy-4-methylpentyl)-3-cylohexene-1-carboxaldehyde, 7-methoxy-3,7-dimethyloctane-1-al, 2-methyldecanal, 1-nonanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methanoindane-1- or -2-carboxaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexene carboxaldehyde, 7-hydroxy-3,7-dimethyl-octanal, trans-4-decenal, 2,6-nonadienal, para-tolylacetaldehyde, 4-methylphenylacetaldehyde, 2-methyl-4-(2,6,6-trimethyl-1-cyclohexen-1-yl)-2-butenal, ortho-methoxycinnamaldehyde, 3,5,6-trimethyl-3-cyclohexene carboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanoindan-1-carboxaldehyde, 2-methyloctanal, alpha-methyl-4-(1-methylethyl)benzolacetaldehyde, 6,6-dimethyl-2-norpinen-2-propionaldehyde, para-methylphenoxyacetaldehyde, 2-methyl-3-phenyl-2-propen-1-al, 3,5,5-trimethylhexanal, hexahydro-8,8-dimethyl-2-naphthaldehyde, 3-propylbicyclo[2.2.1]-hept-5-en-2-carbaldehyde, 9-decenal, 3-methyl-5-phenyl-1-pentanal, floral (4,8-dimethyl-4,9-decadienal), aldehyde C12MNA (2-methylundecanal), liminal (beta-4-dimethylcyclohex-3-ene-1-propan-1-al), methylnonylacetaldehyde, hexanal, trans-2-hexenal and mixtures thereof;
and
wherein the fragrance ketone is selected from methyl-beta-naphthyl ketone, musk indanone (1,2,3,5,6,7-hexahydro-1,1,2,3,3-pentamethyl-4H-inden-4-one), calone (methylbenzodioxepinone), tonalide (6-acetyl-1,1,2,4,4,7-hexamethyltetraline), alpha-damascone, beta-damascone, delta-damascone, iso-damascone, damascenone, methyl dihydrojasmonate (hedione), menthone, carvone, camphor, Koavone (3,4,5,6,6-pentamethylhept-3-en-2-one), fenchone, alpha-ionone, beta-ionone, dihydro-beta-ionone, gamma-methyl-ionone, Fleuramone (2-heptyl cyclopentanone), dihydrojasmone, cis-jasmone, 1-(1,2,3,4,5,6,7,8-octahydro-2,3,8,8-tetramethyl-2-naphthalenyl)-ethan-1-one and isomers thereof, methyl cedrenyl ketone, methylacetophenone, para-methylacetophenone, methyl-beta-naphtylketone, benzylacetone, para-hydroxyphenylbutanone, celery ketone(3-methyl-5-propyl-2-cyclohexenone), 6-isopropyldeca-hydro-2-naphtone, dimethyloctenone, Freskomenthe (2-butan-2-ylcyclohexan-1-one), 4-(1-ethoxyvinyl)-3,3,5,5-tetramethylcyclohexanone, methylheptenone, 2-(2-(4-methyl-3-cyclohexen-1-yl)propyl)cyclopentanone, 1-(p-menthen-6(2)yl)-1-propanone, 4-(4-hydroxy-3-methoxyphenyl)-2-butanone, 2-acetyl-3,3-dimethyl norbornane, 6,7-dihydro-1,1,2,3,3-pentamethyl-4(5H)in-danone, 4-damascol, dulcinyl (4-(1,3-benzodioxol-5-yl)butan-2-one), hexalone (1-(2,6,6-trimethyl-2-cyclohexen-1-yl)-1,6-heptadien-3-one), isocyclemone E 2-acetonaphthon-1,2,3,3,4,5,6,7,8-octahydro-2,3,8,8-tetramethyl), methyl nonyl ketone, methyl cyclocitrone, methyl lavendel ketone, orivone (4-tert-amylcyciohexanone), 4-tert-butyl cyclohexanone, delphone (2-pentyl cyclopentanone), muscone (CAS 541 -91 -3), neobutenone (1-(5,5-dimethyl-1-cyclo-hexenyl)pent-4-en-1-one), plicatone (CAS 41724-19-0), veloutone (2,2,5-trimethyl-5-pentylcyclopentan-1-one), 2,4,4,7-tetramethyl-oct-6-en-3-one, tetrameran (6,10-dimethyl undecen-2-one) and mixtures thereof.

2. The fragrance precursor compounds according to claim 1, wherein the compounds are obtained by the reaction of an epoxide, preferably methyl glycidate, with at least one fragrance aldehyde or ketone selected from dihydro-beta-ionone, frambinone methyl ether, hedione, benzylacetone, calone, decanal, cyclamenaldehyde, melonal or mixtures thereof.

3. insect repellent precursor compounds of formula (VI) 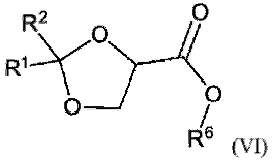 where
R¹ and R² are independently selected from H or linear, branched or cyclic, saturated or unsaturated, substituted or unsubstituted hydrocarbon groups, which contain up to 20 carbon atoms and optionally up to 6 heteroatoms, or R¹ and R² together with the carbon atom to which they are bonded form a cyclic, saturated or unsaturated, hydrocarbon ring with up to 12 carbon atoms, which may contain up to 6 heteroatoms; and R⁶ is selected from a branched or linear alkyl group with up to 12 carbon atoms, particularly preferably methyl, ethyl or propyl;
with the proviso that the group R¹-C-R² is derived from an insect repellent aldehyde of formula R¹-C(=O)H or an insect repellent ketone of formula R¹-C(=O)-R²;
wherein the insect repellent aldehyde is callicarpenal,
wherein the insect repellent ketone is a ketone selected from the group consisting of 2-undecanone, allethrins, terpene ketones, nootkatone and mixtures thereof.

4. A washing or cleaning agent containing at least one fragrance precursor compound according to one of claims 1 or 2, wherein the compound is preferably contained in a total amount of between 0.001 and 5 wt.%, advantageously between 0.005 and 4 wt.%, more advantageously between 0.01 and 2 wt.%, in each case based on the total weight of the agent.

5. The washing or cleaning agent according to claim 4, **characterized in that**
(1) it contains at least one surfactant selected from anionic, cationic, nonionic, zwitterionic, amphoteric surfactants or mixtures thereof;
and/or
(2) it is present in solid or liquid form.

6. An air care agent containing at least one fragrance precursor compound according to one of claims 1 or 2, wherein the compound is preferably contained in a total amount of between 0.001 and 5 wt.%, advantageously between 0.005 and 4 wt.%, more advantageously between 0.01 and 2 wt.%, based on the total weight of the agent.

7. A cosmetic agent containing at least one fragrance precursor compound according to one of claims 1 or 2, wherein the compound is preferably contained in a total amount of between 0.001 and 5 wt.%, advantageously between 0.005 and 4 wt.%, more advantageously between 0.01 and 2 wt.%, based on the total weight of the agent.

8. An insect repellent agent containing at least one insect repellent precursor compound according to claim 3, wherein the compound is preferably contained in a total amount of between 1 and 99 wt.%, preferably between 2 and 50 wt.%, more preferably between 10 and 20 wt.%, based on the total weight of the agent.

9. A process for producing fragrance precursor compounds of formula (III) as defined in claim 1, comprising reacting a compound according to general formula (VII) 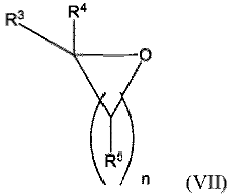 where
R³ is H,
R⁴ is an ester group of formula -C(=O)-O-R⁶,
R⁵ is H,
n corresponds to 1, and
R⁶ is as defined in formula (III) of claim 1,
with aldehydes of formula R¹-C(=O)H or ketones of formula R¹-C(=O)-R² or mixtures thereof, where R¹ and R² are as defined in formula (III).

10. A process for producing insect repellent precursor compounds of formula (VI) as defined in claim 3, comprising reacting a compound according to general formula (VII) 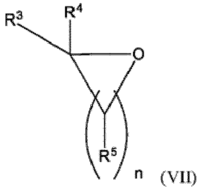 where
R³ is H,
R⁴ is an ester group of formula -C(=O)-O-R⁶,
R⁵ is H,
n corresponds to 1, and
R⁶ is as defined in formula (VI) of claim 3,
with aldehydes of formula R¹-C(=O)H or ketones of formula R¹-C(=O)-R² or mixtures thereof, where R¹ and R² are as defined in formula (VI).

11. A process for long-lasting fragrancing of surfaces, **characterized in that** a fragrance precursor compound according to one of claims 1 or 2 or an agent according to claims 4, 5 or 7 is applied to the surface to be fragranced, the fragrancing lasting longer than would the relevant fragrance compound or an identical agent in which the fragrance precursor compound is replaced by the relevant fragrance compound.

## Revendications

1. Composés précurseurs de substance odoriférante de formule (III) 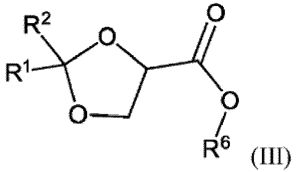 où
R¹ et R² sont choisis, indépendamment l'un de l'autre, parmi H ou des radicaux hydrocarbonés linéaires, ramifiés ou cycliques, saturés ou insaturés, substitués ou non substitués, qui contiennent jusqu'à 20 atomes de carbone et, le cas échéant, jusqu'à 6 hétéroatomes, ou R¹ et R² forment, conjointement avec l'atome de carbone auquel ils sont liés, un noyau hydrocarboné cyclique, saturé ou insaturé, comportant jusqu'à 12 atomes de carbone et qui peut contenir jusqu'à 6 hétéroatomes ; et R⁶ est choisi parmi des radicaux hydrocarbonés linéaires, ramifiés ou cycliques, saturés ou insaturés, substitués ou non substitués, qui contiennent jusqu'à 20 atomes de carbone et, le cas échéant, jusqu'à 6 hétéroatomes ;
à condition que le radical R¹-C-R² soit dérivé d'un aldéhyde de substance odoriférante de formule R¹-C(=O)H ou d'une cétone de substance odoriférante de formule R¹-C(=O)-R² ;
l'aldéhyde de substance odoriférante étant choisi parmi l'Adoxal (2,6,10-triméthyl-9-undécénal), le Cymal ou l'aldéhyde de cyclamen (3-(4-isopropylphényl)-2-méthylpropanal), le Nympheal (3-(4-isobutyl-2-méthylphényl)propanal), l'éthylvanilline, le Florhydral (3-(3-isopropylphényl)butanal]), le Trifernal (3-phénylbutyraldéhyde), l'Helional (3-(3,4-méthylènedioxyphényl)-2-méthylpropanal), l'héliotropine, l'hydroxycitronellal, l'aldéhyde laurique, le Lyral (3- et 4-(4-hydroxy-4-méthylpentyl)-3-cyclohexène-1-carboxaldéhyde), le méthylnonylacétaldéhyde, le Lilial (3-(4-tert-butylphényl)-2-méthylpropanal), le phénylacétaldéhyde, l'aldéhyde undécylénique, la vanilline, le 2,6,10-triméthyl-9-undécénal, le 3-dodécén-1-al, l'aldéhyde alpha-n-amylcinnamique, le Melonal (2,6-diméthyl-5-hepténal), le Triplal (2,4-diméthyl-3-cyclohexène-1-carboxaldéhyde), le 3-(4-tert-butylphényl)-propanal, le 2-méthyl-3-(para-méthoxyphényl)propanal, le 2-méthyl-4-(2,6,6-triméthyl-2(1)-cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, l'aldéhyde 4-isopropylbenzylique, le 1,2,3,4,5,6,7,8-octahydro-8,8-diméthyl-2-naphtaldéhyde, le 2,4-diméthyl-3-cyclohexène-1-carboxaldéhyde, le 2-méthyl-3-(isopropylphényl)propanal, le 1-décanal, le 2,6-diméthyl-5-hepténal, le 4-(tricyclo[5.2.1.0(2,6)]-décylidène-8)-butanal, l'octahydro-4,7-méthane-1H-indènecarboxaldéhyde, le 3-éthoxy-4-hydroxybenzaldéhyde, l'aldéhyde para-éthyl-alpha,alpha-diméthylhydrocinnamique, l'aldéhyde alpha-méthyl-3,4-(méthylènedioxy)-hydrocinnamique, le 3,4-méthylènedioxybenzaldéhyde, l'aldéhyde alpha-n-hexylcinnamique, le m-cymène-7-carboxaldéhyde, l'alpha-méthylphénylacétaldéhyde, le Tétrahydrocitral (3,7-diméthyloctanal), l'undécénal, le 2,4,6-triméthyl-3-cyclohexène-1-carboxaldéhyde, le 4-(3)(4-méthyl-3-pentényl)-3-cyclohexènecarboxaldéhyde, le 1-dodécanal, le 2,4-diméthylcyclohexène-3-carboxaldéhyde, le 4-(4-hydroxy-4-méthylpentyl)-3-cylohexène-1-carboxaldéhyde, le 7-méthoxy-3,7-diméthyloctan-1-al, le 2-méthyldécanal, le 1-nonanal, le 2,6,10-triméthyl-5,9-undécadiénal, le 2-méthyl-3-(4-tert-butyl)propanal, l'aldéhyde dihydrocinnamique, le 1-méthyl-4-(4-méthyl-3-pentényl)-3-cyclohexène-1-carboxaldéhyde, le 5- ou 6-méthoxyhexahydro-4,7-méthanindane-1- ou -2-carboxaldéhyde, le 3,7-diméthyloctan-1-al, le 1-undécanal, le 10-undécén-1-al, le 4-hydroxy-3-méthoxybenzaldéhyde, le 1-méthyl-3-(4-méthylpentyl)-3-cyclohexènecarboxaldéhyde, le 7-hydroxy-3,7-diméthyl-octanal, le trans-4-décénal, le 2,6-nonadiénal, le para-toluylacétaldéhyde, le 4-méthylphénylacétaldéhyde, le 2-méthyl-4-(2,6,6-triméthyl-1-cyclohexén-1-yl)-2-buténal, l'aldéhyde ortho-méthoxycinnamique, le 3,5,6-triméthyl-3-cyclohexènecarboxaldéhyde, le 3,7-diméthyl-2-méthylén-6-octénal, le phénoxyacétaldéhyde, le 5,9-diméthyl-4,8-décadiénal, l'aldéhyde de pivoine (6,10-diméthyl-3-oxa-5,9-undécadién-1-al), l'hexahydro-4,7-méthanindane-1-carboxaldéhyde, le 2-méthyloctanal, l'alpha-méthyl-4-(1-méthyléthyl)benzèneacétaldéhyde, le 6,6-diméthyl-2-norpinène-2-propionaldéhyde, le para-méthylphénoxyacétaldéhyde, le 2-méthyl-3-phényl-2-propén-1-al, le 3,5,5-triméthylhexanal, l'hexahydro-8,8-diméthyl-2-naphtaldéhyde, le 3-propylbicyclo[2.2.1]-hept-5-ène-2-carbaldéhyde, le 9-décénal, le 3-méthyl-5-phényl-1-pentanal, le Floral (4,8-diméthyl-4,9-décadiénal), l'aldéhyde C12MNA (2-méthylundécanal), le Liminal (bêta-4-diméthylcyclohex-3-ène-1-propan-1-al), le méthylnonylacétaldéhyde, l'hexanal, le trans-2-hexénal et leurs mélanges ; et la cétone de substance odoriférante étant choisie parmi la méthyl-bêta-naphtylcétone, l'indanone de Moschus (1,2,3,5,6,7-hexahydro-1,1,2,3,3-pentaméthyl-4H-indén-4-one), la Calone (méthylbenzodioxépinone), la Tonalide (6-acétyl-1,1,2,4,4,7-hexaméthyltétraline), l'alpha-damascone, la bêta-damascone, la delta-damascone, l'iso-damascone, la damascénone, le méthyldihydrojasmonate (Hédione), la menthone, la carvone, le camphre, la Koavone (3,4,5,6,6-pentaméthylhept-3-én-2-one), la fenchone, l'alpha-ionone, la bêta-ionone, la dihydro-bêta-ionone, la gamma-méthyl-ionone, la Fleuramone (2-heptylcyclopentanone), le méthyléther de Frambinone (4-(4-méthoxyphényl)butan-2-one), la dihydrojasmone, la cis-jasmone, la 1-(1,2,3,4,5,6,7,8-octahydro-2,3,8,8-tétraméthyl-2-naphtalényl)-éthan-1-one et ses isomères, la méthylcédrénylcétone, la méthylacétophénone, la para-méthoxyacétophénone, la méthyl-bêta-naphtylcétone, la benzylacétone, la para-hydroxyphénylbutanone, la cétone de céleri (3-méthyl-5-propyl-2-cyclohexénone), la 6-isopropyldéca-hydro-2-naphtone, la diméthylocténone, la Frescomenthe (2-butan-2-ylcyclohexan-1-one), la 4-(1-éthoxyvinyl)-3,3,5,5-tétraméthylcyclohexanone, la méthylhepténone, la 2-(2-(4-méthyl-3-cyclohexén-1-yl)propyl)cyclopentanone, la 1-(p-menthén-6(2)yl)-1-propanone, la 4-(4-hydroxy-3-méthoxyphényl)-2-butanone, le 2-acétyl-3,3-diméthylnorbornane, la 6,7-dihydro-1,1,2,3,3-pentaméthyl-4(5H)in-danone, le 4-damascol, le Dulcinyl (4-(1,3-benzodioxol-5-yl)butan-2-one), l'Hexalon (1-(2,6,6-triméthyl-2-cyclohexén-1-yl)-1,6-heptadién-3-one), l'Isocyclemone E (2-acétonaphthon-1,2,3,4,5,6,7,8-octahydro-2,3,8,8-tétraméthyle), la méthylnonylcétone, la méthylcyclocitrone, la cétone de méthyl-lavande, l'Orivone (4-tert-amylcyclohexanone), la 4-tert-butylcyclohexanone, la Delphone (2-pentylcyclopentanone), la Muscone (CAS 541-91-3), la Néobuténone (1-(5,5-diméthyl-1-cyclo-hexényl)pent-4-én-1-one), la Plicatone (CAS 41724-19-0), la Veloutone (2,2,5-triméthyl-5-pentylcyclopentan-1-one), la 2,4,4,7-tétraméthyl-oct-6-én-3-one, le Tetrameran (6,10-diméthylundécén-2-one) et leurs mélanges.

2. Composés précurseurs de substance odoriférante selon la revendication 1, les composés étant obtenus par transformation d'un époxyde, de préférence du glycidate de méthyle, avec au moins un aldéhyde ou une cétone de substance odoriférante, choisi parmi la dihydro-bêta-ionone, le méthyléther de Frambinone, l'Hédione, la benzylacétone, la Calone, le décanal, l'aldéhyde de cyclamen, le Melonal ou leurs mélanges.

3. Composés précurseurs d'agent insectifuge de formule (VI) 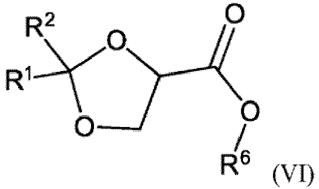 où
R¹ et R² sont choisis, indépendamment l'un de l'autre, parmi H ou des radicaux hydrocarbonés linéaires, ramifiés ou cycliques, saturés ou insaturés, substitués ou non substitués, qui contiennent jusqu'à 20 atomes de carbone et, le cas échéant, jusqu'à 6 hétéroatomes, ou R¹ et R² forment, conjointement avec l'atome de carbone auquel ils sont liés, un noyau hydrocarboné cyclique, saturé ou insaturé, comportant jusqu'à 12 atomes de carbone et qui peut contenir jusqu'à 6 hétéroatomes ; et R⁶ est choisi parmi un radical alkyle ramifié ou linéaire comportant jusqu'à 12 atomes de carbone, de manière particulièrement préférée méthyle, éthyle ou propyle ;
à condition que le radical R¹-C-R² soit dérivé d'un aldéhyde d'agent insectifuge de formule R¹-C(=O)H ou d'une cétone d'agent insectifuge de formule R¹-C(=O)-R² ;
l'aldéhyde d'agent insectifuge étant le Callicarpenal,
la cétone d'agent insectifuge étant une cétone choisie dans le groupe constitué de 2-undécanone, alléthrines, cétones terpéniques, Nootkatone et leurs mélanges.

4. Agent de lavage ou de nettoyage contenant au moins un composé précurseur de substance odoriférante selon l'une des revendications 1 ou 2, le composé étant contenu de préférence en une quantité totale allant de 0,001 à 5 % en poids, avantageusement de 0,005 à 4 % en poids, plus avantageusement de 0,01 à 2 % en poids, respectivement par rapport au poids total de l'agent.

5. Agent de lavage ou de nettoyage selon la revendication 4,
**caractérisé en ce que**
(1) il contient au moins un tensioactif choisi parmi les tensioactifs anioniques, cationiques, non ioniques, zwitterioniques, amphotères ou leurs mélanges ;
et/ou
(2) il se trouve sous forme solide ou liquide.

6. Agent de traitement de l'air contenant au moins un composé précurseur de substance odoriférante selon l'une des revendications 1 ou 2, le composé étant contenu de préférence en une quantité totale allant de 0,001 à 5 % en poids, avantageusement de 0,005 à 4 % en poids, plus avantageusement de 0,01 à 2 % en poids, par rapport au poids total de l'agent.

7. Agent cosmétique contenant au moins un composé précurseur de substance odoriférante selon l'une des revendications 1 ou 2, le composé étant contenu de préférence en une quantité totale allant de 0,001 à 5 % en poids, avantageusement de 0,005 à 4 % en poids, plus avantageusement de 0,01 à 2 % en poids, par rapport au poids total de l'agent.

8. Agent insectifuge contenant au moins un composé précurseur d'agent insectifuge selon la revendication 3, le composé étant contenu de préférence en une quantité totale allant de 1 à 99 % en poids, de préférence de 2 à 50 % en poids, plus préférablement de 10 à 20 % en poids, par rapport au poids total de l'agent.

9. Procédé pour la préparation de composés précurseurs de substance odoriférante de formule (III) tels que définis dans la revendication 1, comprenant la transformation d'un composé selon la formule générale (VII) 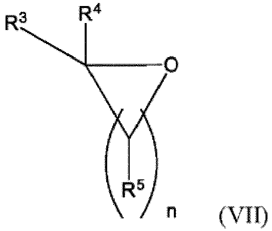 où
R³ représente H,
R⁴ représente un radical ester de formule -C(=O)-O-R⁶,
R⁵ représente H,
n correspond à 1, et
R⁶ est défini comme dans la formule (III) de la revendication 1,
avec des aldéhydes de formule R¹-C(=O)H ou des cétones de formule R¹-C(=O)-R² ou leurs mélanges, R¹ et R² étant définis comme dans la formule (III).

10. Procédé pour la préparation de composés précurseurs d'agent insectifuge de formule (VI) tels que définis dans la revendication 3, comprenant la transformation d'un composé selon la formule générale (VII) 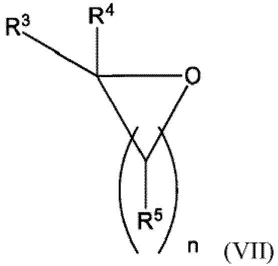 où
R³ représente H,
R⁴ représente un radical ester de formule -C(=O)-O-R⁶,
R⁵ représente H,
n correspond à 1, et
R⁶ est défini comme dans la formule (VI) de la revendication 3,
avec des aldéhydes de formule R¹-C(=O)H ou des cétones de formule R¹-C(=O)-R² ou leurs mélanges, R¹ et R² étant définis comme dans la formule (VI).

11. Procédé destiné à parfumer de manière durable des surfaces,
**caractérisé en ce qu'**un composé précurseur de substance odoriférante selon l'une des revendications 1 ou 2 ou un agent selon la revendication 4, 5 ou 7 est appliqué sur la surface à parfumer, le parfum durant plus longtemps que si le composé de substance odoriférante respectif ou un agent identique, dans lequel le composé précurseur de substance odoriférante est remplacé par le composé de substance odoriférante respectif, avait été utilisé.
